# EUROPÄISCHE PATENTSCHRIFT

(11) **EP 2 438 415 B1**
(45) Veröffentlichungstag und Bekanntmachung des Hinweises auf die Patenterteilung: **03.05.2017**
(21) Anmeldenummer: 10722635.9
(22) Anmeldetag: 01.06.2010
(51) Int. Cl.: G01K 13/12

(54) **EINWURFSONDE**
INSERTION PROBE
SONDE À IMMERSION

(30) Priorität: 05.06.2009 DE 102009024265; 18.12.2009 DE 102009059780
(43) Veröffentlichungstag der Anmeldung: 11.04.2012
(73) Patentinhaber: Heraeus Electro-Nite International N.V., 3530 Houthalen (BE)
(72) Erfinder: BEYENS, Dries, B-3640 Kinrooi (BE)
(74) Vertreter: Heraeus IP
(86) Internationale Anmeldenummer: PCT/EP2010/003310
(87) Internationale Veröffentlichungsnummer: WO 2010/139453

(56) Entgegenhaltungen:
- US-A- 3 463 005
- US-A- 3 577 886
- US-A- 3 766 772

## Beschreibung

Die Erfindung betrifft eine Einwurfsonde zum Bestimmen von Phasenübergängen einer aus einer Stahlschmelze entnommenen Probe durch thermische Analyse, mit einem ein Eintauchende aufweisenden Messkopf, in dem eine eine Einlauföffnung aufweisende Probenkammer und ein mit seiner heißen Lötstelle in die Probenkammer hineinragendes Thermoelement angeordnet sind und der eine Kabeldurchführung für Signalkabel des Thermoelementes aufweist, wobei die Kabeldurchführung an einem dem Eintauchende gegenüberliegenden Ende des Messkopfes aus diesem aus einer Austrittsöffnung austritt und wobei eine Gerade zwischen dem Eintauchende und der Austrittsöffnung eine Längsachse des Messkopfes bildet.

Derartige Sonden sind beispielsweise aus US 3,463,005 bekannt. Hier ist eine Sonde beschrieben, die aus größerer Höhe an einem Signalkabel in eine Metallschmelze abgelassen wird. Zur Stabilisierung weist der Messkopf ein an dessen dem Eintauchende abgewandten Ende ein Papprohr auf, durch das hindurch das Signalkabel geführt wird. Am Eintauchende der Sonde ist ein Thermoelement angeordnet. Ein weiteres Thermoelement ist seitlich in einer aus feuerfestem Material gebildeten Kammer angeordnet und dient der Bestimmung der Liquiduskurve. Eine weitere Einwurfsonde ist aus US 4,881,824 bekannt. Sie weist an ihrem Frontende ein Thermoelement sowie seitlich eine Probennahmekammer auf. Eine ähnliche Einwurfsonde ist in US 5,275,488 beschrieben. Bei dieser Sonde ist ein aus dem Messkopf herausragendes Thermoelement an dessen Eintauchende angeordnet. Es ist durch Metallstreben geschützt, die eine Art Käfig am Eintauchende der Sonde bilden.

Eintauchsonden, die mittels eines Trägerrohres, das auf eine sogenannte Lanze aufgesteckt werden kann, in die Schmelze eingetaucht werden, sind beispielsweise aus US 4,842,418 oder US 5,577,841 bekannt. Die dort beschriebenen Vorrichtungen weisen jeweils an ihrer Frontseite eine Probenkammer auf. Eine weitere Eintauchsonde ist in DE 3919362 A1 beschrieben. Hier ist in einem Trägerrohr eine Probenkammer zur Messung der Liquidustemperatur angeordnet.

Ein Sensor zur Bestimmung des Kohlenstoffgehaltes von Stahlschmelzen in Konverteröfen ist aus CN 201041556 Y bekannt.

Aufgabe der vorliegenden Erfindung ist es, eine verbesserte Einwurfsonde bereit zu stellen, um genauere Messungen, insbesondere in einem Konverter zu ermöglichen.

Die Aufgabe wird durch eine Einwurfsonde gemäß Anspruch 1 gelöst. Vorteilhafte Ausgestaltungen ergeben sich aus den Unteransprüchen. Dadurch, dass der Messkopf zwischen seinem Eintauchende und einer zwischen dem Eintauchende und der Austrittsöffnung die Gerade senkrecht schneidenden Ebene eine Dichte von mindestens 7 g/cm³ aufweist, dass die Einlauföffnung zwischen dieser Ebene und dem Eintauchende oder an dem Eintauchende mündet und dass die Gesamtdichte des Messkopfes weniger als 7 g/cm³ beträgt, vorzugsweise dass senkrecht zur Längsachse jeweils eine virtuelle Ebene durch die heiße Lötstelle und durch eine von dem Eintauchende am weitesten entfernt angeordneten Teil der Einlauföffnung gebildet ist und dass a) entweder der Messkopf zwischen dem Eintauchende und der von dem Eintauchende am weitesten entfernten Ebene eine Dichte von mindestens 7 g/cm³ aufweist oder dass b) der Messkopf zwischen dem Eintauchende und einer Hilfsebene, die im Abstand von mindestens 10 mm von der von dem Eintauchende am weitesten entfernten Ebene parallel zu dieser gebildet ist, eine Dichte von 7 g/cm³ aufweist, kann die Sonde die auf der Metallschmelze aufliegende Schlacke in vertikaler Position durchdringen und vertikal in die Metallschmelze eintauchen. Die Hilfsebene ist vorzugsweise zwischen der von dem Eintauchende am weitesten entfernten Ebene und dem Eintauchende angeordnet.

Mit der erfindungsgemäßen Eintauchsonde ist es möglich, den Kohlenstoffgehalt der Stahlschmelze in einem Konverter (BOF-Kessel) zu analysieren durch Messung der Liquidustemperatur. Dies kann während des Blasvorganges im Konverter erfolgen. Gleichzeitig kann die Temperatur der Stahlschmelze gemessen werden. Durch die Möglichkeit, die Messungen während des Blasvorganges vorzunehmen, kann der gesamte Prozess optimiert werden, so dass verhindert wird, dass zu viel Gas in die Schmelze eingeblasen wird. Die Sonde dringt dazu nahezu senkrecht in die Metallschmelze ein. Der Messkopf kann aus mehreren in Eintauchrichtung hintereinander angeordneten Stahlkörpern bestehen.

Der Messkopf ist ein im Wesentlichen aus Metall, vorzugsweise Stahl gebildeter Körper, der sich zwischen dem Eintauchende und dem Kabelaustritt erstreckt. Der Messkopf umfasst neben seinem Metallkörper das Volumen und Material des Thermoelementes, der Probenkammer und der Einlauföffnung. Letzere ist als Rohr ausgebildet. Die Form des Metallkörpers ist definiert durch seine Silhouette, wobei die Einlauföffnung der Probenkammer und die Auslassöffnung des Signalkabels als geschlossen betrachtet werden.

Es ist vorteilhaft, dass die Einlauföffnung einen Durchmesser aufweist, der mindestens ein Drittel des Durchmessers der Probenkammer beträgt und höchstens so groß ist wie der Durchmesser der Probenkammer.
Der Abstand der Hilfsebene von der vom Eintauchende am weitesten entfernten Ebene beträgt zweckmäßigerweise mindestens 20 mm, insbesondere mindestens 30 mm. Die Hilfsebene liegt vorzugsweise zwischen dem Eintauchende und der vom Eintauchende am weitesten entfernten Ebene. Dadurch liegt der Schwerpunkt der Eintauchsonde relativ weit vorn am Eintauchende des Messkopfes.

Das Thermoelement erstreckt sich vorzugsweise etwa parallel zur Längsachse mit seiner heißen Lötstelle in Richtung des Eintauchendes. Zweckmäßig ist es weiterhin, dass das Thermoelement mit einem gasdurchlässigen Material in der Probenkammer gehalten ist. Die Kabeldurchführung und die Austrittsöffnung über das gasdurchlässige Material ist zweckmäßigerweise ebenfalls gasdurchlässig mit der Probenkammer verbunden, so dass Gase aus der Probenkammer nahezu ungehindert austreten können, insbesondere ist es auch von Vorteil, dass an der Austrittsöffnung ein vorzugsweise koaxial zu der Geraden verlaufendes Rohr, vorzugsweise ein Metallrohr angeordnet ist, durch das die Signalkabel geführt sind. Das Metallrohr erstreckt sich dabei insbesondere in Richtung der Längsachse des Messkopfes. Es dient der zusätzlichen Stabilisierung der Messsonde während des Einwurfes und des zeitweiligen Schutzes der Signalkabel vor der vorzeitigen Zerstörung in der Metallschmelze.

Zweckmäßig ist es weiterhin, dass die Einlauföffnung an ihrer Außenseite eine Schutzkappe aufweist, die aus Metall oder einer Kombination aus Metall mit Pappe oder mit Papier gebildet ist. Dadurch wird das vorzeitige Eindringen von Material in die Probenkammer verhindert, bevor die Einlauföffnung unterhalb des Badspiegels der Metallschmelze angelangt ist. Die Schutzkappe ist vorzugsweise aus Stahl gebildet, sie weist zweckmäßigerweise eine Dicke von höchstens 0,5 mm auf ebenso kann die Schutzkappe aus einer Kombination aus Zink mit Pappe oder mit Papier oder aus Stahl mit einer Auflage aus Zink gebildet sein. Die Einlauföffnung ist vorteilhafterweise aus Quarzglas gebildet. In der Einlauföffnung kann ein desoxidierendes Mittel angeordnet sein. Hier ist Aluminium als geeignetes Material beispielhaft zu nennen.

Eine weitere vorteilhafte Ausgestaltung der erfindungsgemäßen Einwurfsonde zeichnet sich dadurch aus, dass der Messkörper im Wesentlichen aus Metall, vorzugsweise Stahl gebildet ist. Alternativ kann der Messkörper auch aus einem Gussstück geformt sein. Dabei bieten sich insbesondere Eisenguss oder Grauguss an. In solcher Art ausgestaltete Messköpfe können dann die weiteren Elemente wie Thermoelemente, Probenkammer etc. integriert werden. Besagte Materialien ermöglichen eine langfristige strukturelle Stabilität des Messkopfes auch in der Stahlschmelze, so dass die in die Probenkammer einfließende Stahlschmelze erstarren und eine zuverlässige sowie reproduzierbare Messung erstellt werden kann. Vorteilhafterweise weist der Probenkörper ein Volumen von wenigstens 450 cm³ auf. In Verbindung mit der erfindungsgemäßen ausgestalteten Gesamtdichte des Messkopfes wird so sichergestellt, dass der Messkopf ausreichend lang funktionssicher in der flüssigen Stahlschmelze arbeiten kann.

Beim Eintauchen des Messkopfes in die Stahlschmelze fließt jene durch eine Einlauföffnung in die Probenkammer des Messkopfes ein. Die Dimension der Probenkammer muss so gewählt werden, dass ein schnelles Abkühlen des geschmolzenen Metalls sichergestellt werden kann. Da nach einer mittleren Zeitdauer von 8 - 10 Sekunden die Verbindungskabel zwischen dem Messkopf und einer Auswerteelektronik durchgeschmolzen werden, muss in dieser Zeitspanne ein reproduzierbares Erstarren des flüssigen Metalls in der Probenkammer erfolgen, um die benötigten Messungen durchzuführen. Als besonders vorteilhaft haben sich Probekammern herausgestellt, welche ein Volumen zwischen 7 und 50 cm³ aufweisen. Um ein gleichmäßiges Einströmen der flüssigen Stahlschmelze in die Probenkammer weist letztere eine Gasaustrittsöffnung auf. Die von der Stahlschmelze verdrängte Luft kann durch jene Gasaustrittsöffnung die Probenkammer verlassen und damit Platz für die nachdrängende Stahlschmelze machen. Jene Gasaustrittsöffnung befindet sich an einer Oberseite des erfindungsgemäßen Messkopfes. Erfindungsgemäss wird die Gasaustrittsöffnung durch einen Spalt gebildet, welcher zwischen dem Signalkabel und einem Metallrohr angeordnet ist. Letzteres Metallrohr schützt dabei das Signalkabel vor einer vorzeitigen Beschädigung durch die Schlacke und/oder die Stahlschmelze.

Die Erfindung zeichnet sich dadurch aus, dass die Gesamtdichte des Messkopfs weniger als 7 g/cm³ beträgt. Dabei werden nur das Volumen sowie das Gewicht des Messkopfes berücksichtigt.

Eine weitere vorteilhafte Ausführungsform der Erfindung zeichnet sich dadurch aus, dass die Gesamtdichte des in die Stahlschmelze und die Schlacke eintauchenden Teils der Einwurfsonde eine Gesamtdichte von weniger als 7 g/cm³ aufweisen. Bei dieser Ausführungsvariante umfasst der Messkopf auch ein das Signalkabel zumindest teilweise umgebenden Metallrohr und Teile des Signalkabels, insofern diese Teil mit in die Stahlschmelze und die Schlacke eintauchen. In einer vorteilhaften Variante dieser Ausführungsform der Erfindung muss die Gesamtdichte: des Messkopfs und 50 cm eines Signalkabel bei einer mittleren Kabeldichte von 1,6 g/cm³ weniger als 7 g/cm³ betragen. Damit bezieht sich die erfindungsgemäße Gesamtdichte in dieser Ausführungsform nicht nur auf den Messkopf der Einwurfsonde an sich, sondern auch auf eine definierte Länge des Signalkabels. Die Länge - 0.5 m - des Signalkabels ergibt sich aus der bevorzugten Messposition des Messkopfs ca. 20 cm unterhalb der Oberfläche der Stahlschmelze. Für die Positionierung des Messkopfs ist auch der Auftrieb des Kabels innerhalb der Schlacke auf der Stahlschmelze zu berücksichtigen. Deren Dicke beträgt im Allgemeinen ca. 30 cm. Daher ist erfindungsgemäß gefordert, dass bei der Berechnung der Gesamtdichte von weniger als 7 g/cm³ auch jenen Teile des Einwurfsonde mit berücksichtigt werden, die in die Stahlschmelze und/oder die Schlacke eingetaucht sind. Bei dieser Ausführungsvariante umfasst der Messkopf auch ein das Signalkabel zumindest teilweise umgebenden Metallrohr. Dort wo das Signalkabel dieses Metallrohr verlässt ist der Kabelaustritt.

Die Einwurfsonde kann einen weiteren Temperatursensor aufweisen, mit dem unabhängig die Badtemperatur bestimmt werden kann. Der weitere Temperatursensor kann unter anderem an dem dem Eintauchende entgegengesetzten Ende des Messkopfes angeordnet sein.

Nachfolgend wird die Erfindung beispielhaft an Hand von Zeichnungen beschrieben. In der Zeichnung zeigt:
Fig.1 den Zusammenhang des Einwurfs einer Einwurfsonde in einen Konverter,
Fig.2 den Schnitt durch den Messkopf einer erfindungsgemäßen Einwurfsonde,
Fig.3 eine weitere Ausführungsform einer erfindungsgemäßen Einwurfsonde,
Fig.4 eine weitere erfindungsgemäße Einwurfsonde mit Fronteinlauf,
Fig.5 eine erfindungsgemäße Einwurfsonde mit Hilfsebene ,
Fig.6 eine weitere erfindungsgemäße Einwurfsonde mit Fronteinlauf und
Fig 7 eine weitere erfindungsgemäße Einwurfsonde

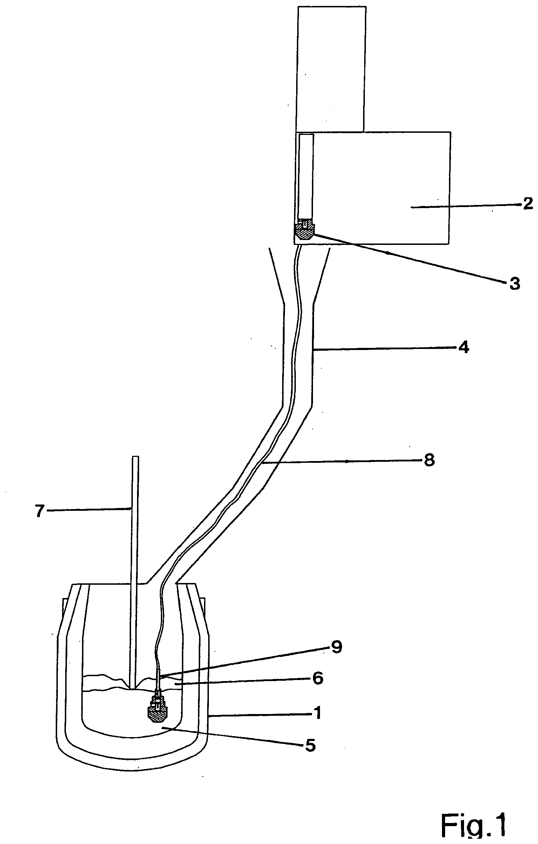

In der in Fig.1 dargestellten Übersicht ist einige Meter oberhalb eines Konverters 1 eine Abwurfvorrichtung 2 angeordnet, die Abwurfvorrichtung 2 dient als Speicher für die Einwurfsonden 3. Die Einwurfsonden 3 werden automatisch aus dem Speicher freigegeben, sie fallen durch eine Führung 4 hindurch in den Konverter 1 und in die darin befindliche Stahlschmelze 5, nachdem sie die über die Stahlschmelze liegende Schlackeschicht 6 durchdrungen haben. In der Figur ist eine Blaslanze 7 dargestellt, mit der Sauerstoff in die Stahlschmelze eingeblasen wird. Die Einwurfsonde 3 ist mit einem Signalkabel 8 verbunden, über welches Messsignale an einen Computer geleitet werden können. Das Signalkabel 8 ist durch ein Metallrohr 9 hindurchgeführt. Das Metallrohr 9 schützt das Signalkabel 8 vor vorzeitiger Beschädigung durch die Schlacke oder Stahlschmelze 5.

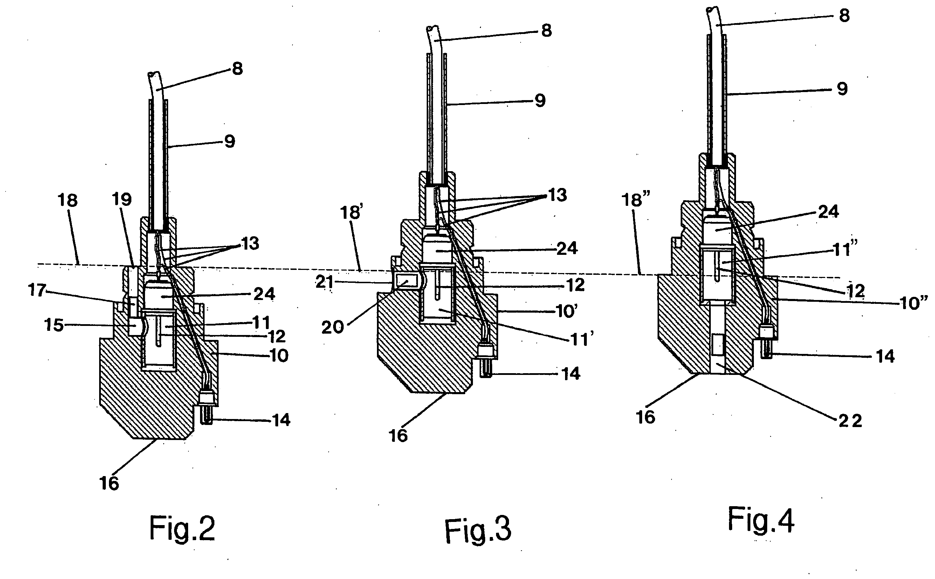

Fig. 2 zeigt einen Einwurfsensor mit Messkopf 10 aus Stahl. Der Messkopf 10 weist eine Probenkammer 11 mit einem Thermoelement 12 auf. Oberhalb der Probenkammer 11 sind die mit dem Signalkabel 8 verbundenen Thermoelementleitungen 13 dargestellt. Über Thermoelementleitungen 13 ist auch ein weiteres, an der Außenseite des Messkopfes 10 angeordnetes Thermoelement 14 mit dem Signalkabel 8 verbunden. Die Einlauföffnung 15 in die Probenkammer 11 endet an der dem Eintauchende 16 des Messkopfes 11 gewandten Seite, so dass der Einlauf in die Probenkammer 11 nach Eintauchen des Messkopfes 10 in die Stahlschmelze 5 von oben erfolgt. In der Einlauföffnung 15 ist ein dünnes Aluminiumblech als desoxidierendes Mittel 17 angeordnet. Zwischen dem Eintauchende 16 und der am oberen Ende der Einlauföffnung 15 angeordneten Ebene 18 beträgt die Dichte des Messkopfes 10 7,0 g/cm³. Die Einlauföffnung 15 ist im Wesentlichen aus einem gebogenen Quarzglasrohr gebildet. Die äußere Öffnung 19 der Einlauföffnung 15, durch welche die Ebene 18 hindurch verläuft, ist mit einer in der Zeichnung nicht dargestellten Schutzkappe aus einem etwa 0,2 bis 0,4 mm dicken Stahlblech gebildet, dass an seiner Außenseite eine dünne Pappschicht aufweist.

In Fig. 3 ist eine ähnliche Einwurfsonde dargestellt. Im Unterschied zu der in Fig. 2 dargestellten Einwurfsonde, ist bei dem in Fig. 3 gezeigten Messkopf 10' der Einlauf 20 in die Probenkammer 11' seitlich angeordnet. Die äußere Öffnung der Einlauföffnung 20 ist mit einer Schutzkappe 21 aus einer mit Pappe beschichteten 0,4 mm dicken Stahlkappe geschlossen. Die Ebene 18' verläuft durch die Oberkante der Einlauföffnung 20. In den Figuren 2 und 3 ist die durch das Oberteil der jeweiligen Einlauföffnung 15; 20 verlaufende virtuelle Ebene 18; 18' weiter von dem Eintauchende 16 entfernt als eine in der Zeichnung nicht dargestellte durch die heiße Lötstelle der jeweiligen Sonde verlaufende virtuelle Ebene.

In der in Fig. 4 dargestellten Ausführungsform einer erfindungsgemäßen Einwurfsonde ist gegenüber den Figuren 2 und 3 der Messkopf 10" mit einer Einlauföffnung 22 versehen, die von der Probenkammer 11" ausgehend in dem Eintauchende 16 des Messkopfes 10" mündet. Hier ist die durch die heiße Lötstelle des Thermoelements 12 verlaufende virtuelle Ebene 18" relevant für die Bestimmung der Dichteverteilung. Die Dichte zwischen der virtuellen Ebene 18" und dem Eintauchende 16 der in Figur 4 darstellten Einwurfsonde beträgt etwa 7,0 g/cm³. In der in Fig. 3 darstellten Ausführungsform beträgt die Dichte zwischen der virtuellen Ebene 18' und dem Eintauchende 16 etwa 7,1 g/cm³.

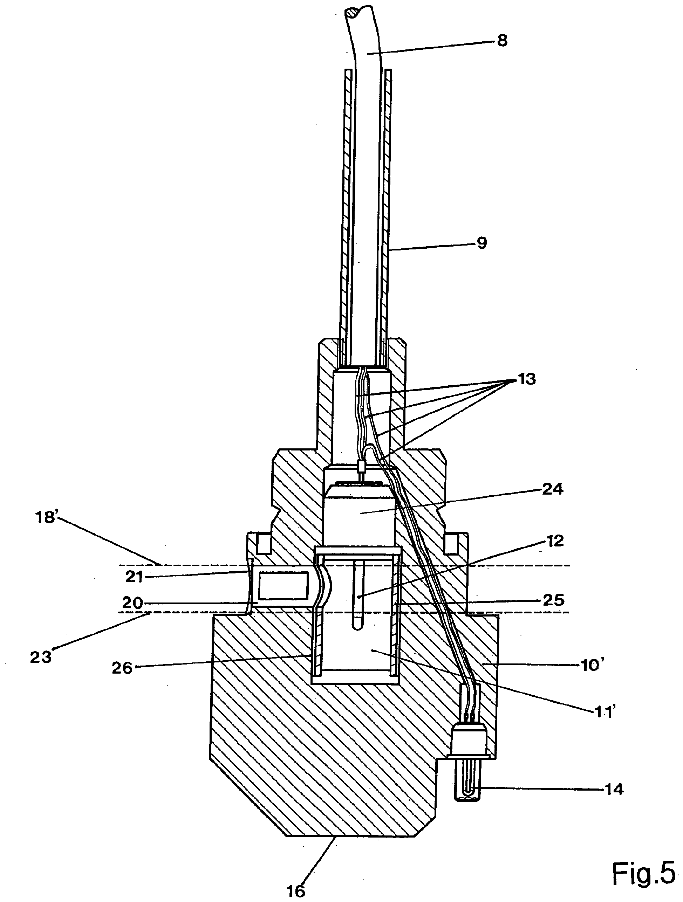

Die in Fig. 5 darstellte Ausführungsform, die prinzipiell der in Fig. 3 dargestellten Ausführungsform ähnelt, wurde so gestaltet, dass die Dichte zwischen einer Hilfsebene 23 und dem Eintauchende 16 des Messkopfes 10' etwa 7,4 g/cm³ beträgt, wobei das Material des Messkopfes 10' im wesentlichen aus Stahl gebildet ist. Die Hilfsebene 23 ist etwa 15 mm unterhalb der virtuellen Ebene 18' (in Richtung des Eintauchendes 16) angeordnet.

Die Gesamtdichte des Messkopfes beträgt jeweils etwas weniger als 6 g/cm³. Der Messkopf ist jeweils etwa 10 cm lang mit einem größten Durchmesser von ebenfalls etwa 10 cm. Das Metallrohr 9 ist jeweils etwa 45 cm lang. Die Signalkabellänge beträgt, abhängig von den Einsatzbedingungen, im Allgemeinen entweder 15 m oder 26 m oder auch 35 m.

Oberhalb, also entfernt von dem Eintauchende 16 ist an der Probenkammer 11; 11'; 11"; 11"' ein Feuerfestmaterial 24 angeordnet, in welchem das Thermoelement 12 fixiert ist.

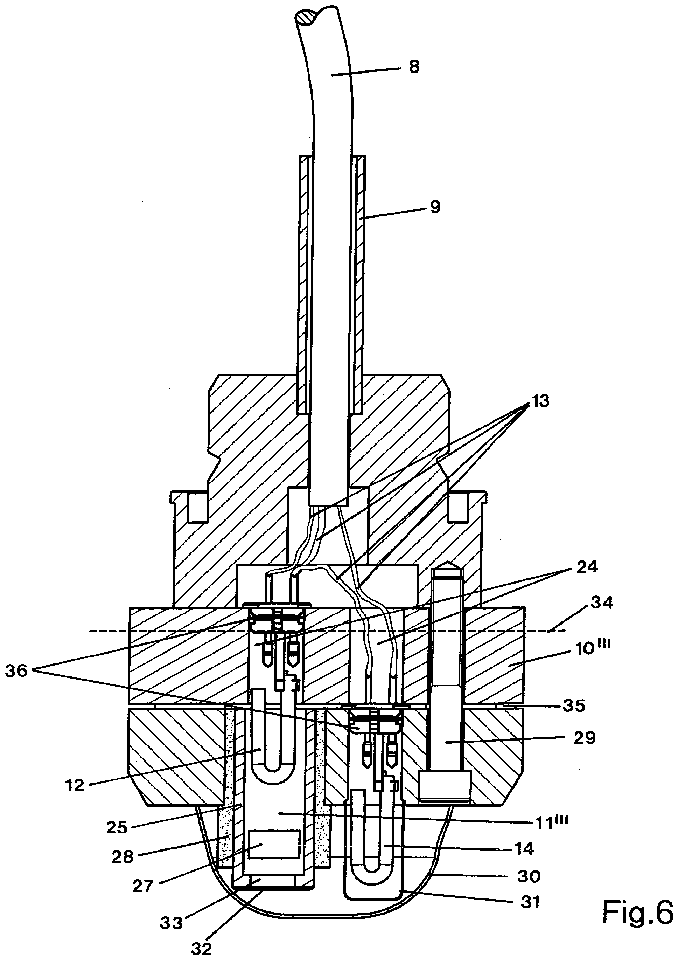

Generell kann an dem Messkopf 10; 10'; 10"; 10'" eine nur in Figur 6 dargestellte Schutzkappe 30 außen angeordnet sein. Diese ist bei der Bestimmung der Dichte nicht zu berücksichtigen.

Das Feuerfestmaterial 24 (beispielsweise Gießereisand oder Zement) ist gasdurchlässig, aber undurchlässig für die Metallschmelze, so das Gase aus der Probenkammer durch das Feuerfestmaterial 24 hindurch dringen können. Sie werden dann durch das Metallrohr 9 nach Außen abgeführt. Die Probenkammer 11; 11'; 11"; 11"' ist, wie in Fig. 5,6 ersichtlich, durch ein isolierendes Material 25 seitlich begrenzt. Zwischen dem isolierendem Material 25 und dem Stahl des Messkopfes 10; 10'; 10"; 10"' ist eine Lücke 26 von etwa 1 mm Breite gebildet. Die Schutzkappe 21 kann auch aus dünnerem (etwa 0,2 mm dickem) Stahl gebildet sein und an der Außenseite eine Schicht aus Pappe, Papier oder Zink aufweisen.

Die in Fig. 6 dargestellte Einwurfsonde ist im Wesentlichen den bereits beschriebenen Sonden ähnlich. Die Probenkammer 11'" ist am Eintauchende des Messkopfes 10'" angeordnet. Die Einlauföffnung 33 weist einen Durchmesser auf, der etwa zwei Drittel des Durchmessers der Probenkammer 11 "' beträgt. Sie ist mit einer Metallkappe 32 geschlossen und mittels Gießereisand 28 in dem Messkopf 10'" befestigt, wobei der Gießereisand die Probenkammer 11"' über den größten Teil ihrer Länge auch Außerhalb des Metallkörpers umgibt. In der Probenkammer 11'" ist ein desoxidierendes Aluminiumstück 27 angeordnet. Neben der Probenkammer 11'" ist ein zweites Thermoelement 14 angeordnet, welches mit einer Metallkappe 31 abgedeckt ist. Beide Thermoelemente 12;14 sind an ihrem rückseitigen Ende mit einem Konnektor 36 verbunden, über den die Verbindung mit den Thermoelementleitungen 13 erfolgt. Zwischen zwei Teilen des Metallkörpers des Messkopfes 10'" sind Entlüftungsöffnungen 35 für die Probenkammer 11"' vorgesehen. Die Entlüftungsöffnungen sichern eine blasenfreie Probe. Sie können als einzelne Bohrungen ausgestaltet sein oder auch als umlaufender Spalt zwischen zwei getrennten Teilen des Metallkörpers. In diesem Fall werden die beiden Teile beispielsweise von Schrauben 29 zusammengehalten. Der vordere Teil des Messkopfes 10"' ist sehr schwer. Zwischen seinem Eintauchende und der Ebene 34 weist er eine Dichte von 7 g/cm³ auf bei einer Gesamtdichte des Messkopfes 10"' von 6,7 g/cm³.

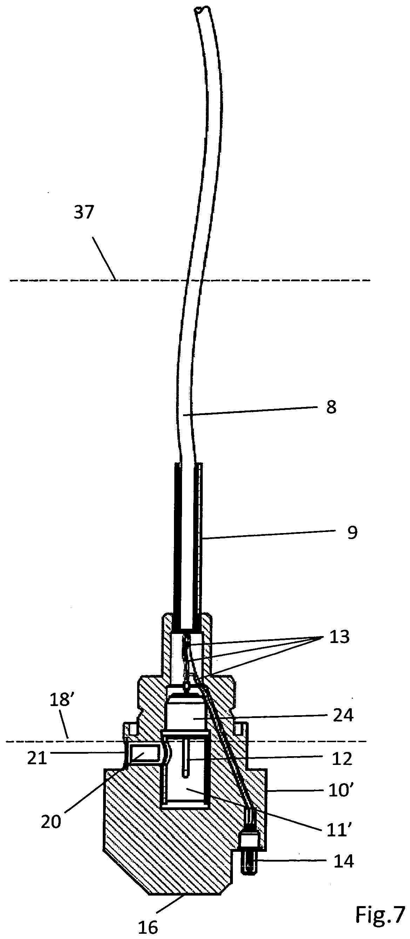

In Fig. 7 ist der in Fig. 3 schon beschriebene Messkopf 10' noch einmal dargestellt. Im Gegensatz zu Messkopf 10' ist nunmehr erfindungsgemäß hervorgehoben, dass die Gesamtdichte des Messkopfes sich ab einer weiteren Ebene 37 ergibt. Ausgangspunkt ist dabei, dass auch ein Teil des Kabels 8 mit in die Stahlschmelze eintaucht und somit bei der Berechnung der Gesamtdichte des Messkopfes berücksichtigt werden muss. Erfindungsgemäß sollte der Messkopf etwa 20 cm unter der Oberfläche des flüssigen Stahles eintauchen und seine Messung vornehmen. Wie schon dargelegt fließt dabei Stahlschmelze durch die Einlauföffnung 19,20,22 in die Probenkammer 11'. Etwaiges Gas kann durch das feuerfeste Material 24 zwischen dem Signalkabel 8 und dem Metallrohr 9 durch eine Austrittsöffnung aus dem Messkopf austreten. Dabei ist es wesentlich, dass die Austrittsöffnung höher angeordnet ist als die Einlauföffnung 19,20,22 der Probenkammer. Im Rahmen der Erfindung bedeutet dabei der Begriff höher, die unterschiedliche Anordnung der Austritts- bzw. Einlauföffnung entlang einer Längsachse des Messkopfes. Dabei ist die Einlauföffnung derart in dem Messkopf angeordnet, dass beim gebrauchsgemäßen Eintauchen des Messkopfes in die Stahlschmelze, jene zuerst in die Einlauföffnung einfließt und/oder ein Befüllen der Probekammer durch die Einlauföffnung erfolgt.

Eine vorteilhafte Ausführungsform der Erfindung zeichnet sich dadurch aus, dass die Gesamtdichte - gebildet aus der Masse und des Volumens des in die Stahlschmelze und die Schlacke eintauchende Teil der gesamten Einwurfsonde - weniger als 7 g/cm³ aufweist. Bei dieser Ausführungsvariante umfasst der Messkopf auch Teile des Signalkabels 8 sowie ein das Signalkabel 8 zumindest teilweise umgebenden Metallrohr 9, insofern diese Bestandteile der Einwurfsonde mit in die Stahlschmelze und die Schlacke eintauchen.

Erfahrungsgemäß weist die Schlacke auf der flüssigen Stahlschmelze eine Dicke von ca. 30 cm auf. Besonders vorteilhaft hat es sich herausgestellt, wenn somit eine Kabellänge von 50 cm-zusammengesetzt aus einer Dicke der Schlacke von ca. 30 cm und dem Probenort von ca. 20 cm bei einer Dichte des Kabels von ca. 1,6 g/cm³ - bei der Berechnung der Gesamtdichte des Messkopfes mit einbezogen wird. In einer vorteilhaften Variante dieser Ausführungsart ergibt sich eine Einwurfsonde bei der die Gesamtdichte des Messkopfs und 50 cm eines Signalkabel 8 bei einer Kabeldichte von 1,6 g/cm³ sowie eine das Signalkabel 8 zumindest teilweise umgebenden Metallrohres 9 weniger als 7 g/cm³ beträgt. So kann sichergestellt werden, dass der Messkopf zuverlässig und reproduzierbar in die Stahlschmelze eintaucht, um entsprechend für die Vermessung der Eigenschaften der Schmelze genutzt zu werden. Somit weist bei dieser Ausführungsform der in die Stahlschmelze und/oder die Schlacke eintauchende Teil der Einwurfsonde eine Gesamtdichte von weniger als 7 g/cm³ auf. Bei dieser Ausführungsvariante umfasst der Messkopf auch ein das Signalkabel zumindest teilweise umgebenden Metallrohr 9. Dort wo das Signalkabel 8 dieses Metallrohr 9 verlässt ist der Kabelaustritt. Bei der Figur 7 dargestellten Ausführungsform ist der Kabelaustritt auch gleichzeitig die Austrittsöffnung - auch als Auslassöffnung bezeichnet.

## Patentansprüche

1. Einwurfsonde zum Bestimmen von Phasenübergängen einer aus einer Stahlschmelze entnommenen Probe durch thermische Analyse, mit einem ein Eintauchende aufweisenden Messkopf (10), in dem eine eine Einlauföffnung (15,20) aufweisende Probenkammer (11) und ein mit seiner heißen Lötstelle in die Probenkammer hineinragendes Thermoelement (12) angeordnet sind und der eine Kabeldurchführung für ein Signalkabel (8) des Thermoelementes aufweist, wobei die Kabeldurchführung an einem dem Eintauchende gegenüberliegenden Ende des Messkopfes aus diesem aus einer Austrittsöffnung austritt und wobei eine Gerade zwischen dem Eintauchende und der Austrittsöffnung eine Längsachse des Messkopfes bildet, wobei der Messkopf zwischen seinem Eintauchende und einer zwischen dem Eintauchende und der Austrittsöffnung die Gerade senkrecht schneidenden Ebene eine Dichte von mindestens 7 g/cm³ aufweist, dass die Einlauföffnung zwischen dieser Ebene und dem Eintauchende oder an dem Eintauchende mündet und dass die Gesamtdichte des Messkopfes weniger als 7 g/cm³ beträgt, **dadurch gekennzeichnet, dass** die Probenkammer eine Gasaustrittsöffnung aufweist, um ein gleichmäßiges Einströmen der flüssigen Stahlschmelze in die Probenkammer sicherzustellen, wobei die Gasaustrittsöffnung so beschaffen ist, dass von der Stahlschmelze verdrängte Luft durch jene Gasaustrittsöffnung die Probenkammer verlassen und damit Platz für die nachdrängende Stahlschmelze machen kann, wobei die Gasaustrittsöffnung durch einen Spalt gebildet wird, welcher zwischen dem Signalkabel (8) und einem Metallrohr (9) angeordnet ist, welches das Signalkabel vor einer vorzeitigen Beschädigung durch die Schlacke und/oder die Stahlschmelze schützt.

2. Einwurfsonde nach Anspruch 1, **dadurch gekennzeichnet, dass** die Gesamtdichte des Messkopfes einschließlich dem das Signalkabel zumindest teilweise umgebenden Metallrohres und Teilen des Signalkabels weniger als 7 g/cm³ beträgt.

3. Einwurfsonde nach Anspruch 1 oder 2, **dadurch gekennzeichnet, dass** senkrecht zur Längsachse jeweils eine virtuelle Ebene durch die heiße Lötstelle und durch einen von dem Eintauchende am weitesten entfernt angeordneten Teil der Einlauföffnung gebildet ist und dass der Messkopf zwischen dem Eintauchende und der von dem Eintauchende am weitesten entfernten Ebene eine Dichte von mindestens 7 g/cm³.

4. Einwurfsonde nacheinem der Ansprüche 1 bis 3, **dadurch gekennzeichnet, dass** die Einlauföffnung einen Durchmesser aufweist, der mindestens ein Drittel des Durchmessers der Probenkammer beträgt und höchstens so groß ist wie der Durchmesser der Probenkammer.

5. Einwurfsonde nach mindestens einem der Ansprüche 1 bis 4, **dadurch gekennzeichnet, dass** das Thermoelement sich etwa parallel zur Längsachse mit seiner heißen Lötstelle in Richtung Eintauchende erstreckt.

6. Einwurfsonde nach mindestens einem der Ansprüche 1 bis 5, **dadurch gekennzeichnet, dass** das Thermoelement mit einem gasdurchlässigen Material in der Probenkammer gehalten ist.

7. Einwurfsonde nach Anspruch 6, **dadurch gekennzeichnet, dass** die Kabeldurchführung und die Austrittsöffnung über das gasdurchlässige Material gasdurchlässig mit der Probenkammer verbunden sind.

8. Einwurfsonde nach mindestens einem der Ansprüche 1 bis 7, **dadurch gekennzeichnet, dass** an der Austrittsöffnung ein vorzugsweise koaxial zu der Geraden verlaufendes Rohr, vorzugsweise ein Metallrohr angeordnet ist, durch das die Signalkabel geführt sind.

9. Einwurfsonde nach mindestens einem der Ansprüche 1 bis 8, **dadurch gekennzeichnet, dass** die Einlauföffnung an ihrer Außenseite eine Schutzkappe aufweist, die aus Metall oder einer Kombination aus Metall mit Pappe oder mit Papier gebildet ist.

10. Einwurfsonde nach Anspruch 9, **dadurch gekennzeichnet, dass** die Schutzkappe aus Stahl, vorzugsweise mit einer Dicke von höchstens 0,5 mm, oder einer Kombination aus Zink mit Pappe oder mit Papier gebildet ist.

11. Einwurfsonde nach mindestens einem der Ansprüche 1 bis 10, **dadurch gekennzeichnet, dass** die Einlauföffnung aus Quarzglas gebildet ist, wobei ein desoxidierendes Mittel in ihr angeordnet sein kann.

12. Einwurfsonde nach mindestens einem der Ansprüche 1 bis 11, **dadurch gekennzeichnet, dass** sie einen weiteren Temperatursensor aufweist.

13. Einwurfsonde nach Anspruch 12, **dadurch gekennzeichnet, dass** der weitere Temperatursensor an dem dem Eintauchende entgegengesetzten Ende des Messkopfes angeordnet ist.

14. Verfahren zum Bestimmen von Phasenübergängen einer aus einer Stahlschmelze entnommenen Probe durch thermische Analyse mit der Einwurfsonde nach einem der vorhergehenden Ansprüche, wobei die Teile des Signalkabels sowie das das Signalkabel (8) zumindest teilweise umgebenden Metallrohr (9), die in die Gesamtdichte des Messkopfes von weniger als 7 g/cm³ einbezogen sind, beim Betrieb der Einwurfsonde in die Stahlschmelze und in eine auf der Stahlschmelze befindliche Schlackeschicht eingetaucht werden.

15. Verfahren nach dem vorhergehenden Anspruch, **dadurch gekennzeichnet, dass** der in die Gesamtdichte des Messkopfes von weniger als 7 g/cm³ einbezogene Teil des Signalkabels 50 cm lang ist bei einer mittleren Kabeldichte von ca. 1,6 g/cm³.

## Claims

1. A drop-in probe for determining the phase transitions of a sample taken from a steel melt by thermal analysis, comprising a measurement head (10) having an immersion end, in which a sample chamber (11) having an inlet opening (15, 20) and a thermocouple (12) extending into the sample chamber with its hot solder location are arranged and which has a cable feedthrough for a signal cable (8) of the thermocouple, wherein the cable feedthrough exits out of said measurement head from an outlet opening at an end of the measurement head located opposite the immersion end, and wherein a straight line between the immersion end and the outlet opening forms a longitudinal axis of the measurement head, wherein the measurement head has a density of at least 7g/cm³ between its immersion end and a plane, which vertically intersects the straight line between the immersion end and the outlet opening, that the inlet opening opens between this plane and the immersion end or at the immersion end and that the overall density of the measurement head is less than 7 g/cm³, **characterised in that** the sample chamber has a gas outlet opening for ensuring an even inflow of the liquid steel melt into the sample chamber, wherein the gas outlet opening is designed in such a way that air displaced by the steel melt can leave the sample chamber through that gas outlet opening and can thus make space for the steel melt, which pushes in, wherein the gas outlet opening is formed by a gap, which is arranged between the signal cable (8) and a metal tube (9), which protects the signal cable against being damaged prematurely by the slag and/or the steel melt.

2. The drop-in probe according to claim 1, **characterised in that** the total density of the measurement head including the metal tube, which at least partially surrounds the signal cable, and parts of the signal cable is less than 7 g/cm³.

3. The drop-in probe according to claim 1 or 2, **characterised in that** a virtual plane is in each case formed vertically to the longitudinal axis through the hot soldering joint and through a part of the inlet opening, which is arranged farthest away from the immersion end, and that the measurement head between the immersion end and the plane, which is farthest away from the immersion end, has a density of at least 7 g/cm³.

4. The drop-in probe according to any one of claims 1 to 3, **characterised in that** the inlet opening has a diameter, which is at least one third of the diameter of the sample chamber and which is maximally as large as the diameter of the sample chamber.

5. The drop-in probe according to at least any one of claims 1 to 4, **characterised in that** the thermocouple extends approximately parallel to the longitudinal axis with its hot solder joint in the direction of the immersion end.

6. The drop-in probe according to at least any one of claims 1 to 5, **characterised in that** the thermocouple is held in the sample chamber with a gas-permeable material.

7. The drop-in probe according to claim 6, **characterised in that** the cable feedthrough and the outlet opening are connected to the sample chamber in a gas-permeable manner via the gas-permeable material.

8. The drop-in probe according to at least any one of claims 1 to 7, **characterised in that** a tube, preferably a metal tube, which preferably runs coaxially to the straight line and through which the signal cables are guided, is arranged on the outlet opening.

9. The drop-in probe according to at least any one of claims 1 to 8, **characterised in that**, on its outer side, the inlet opening has a protective cap, which is formed from metal or a combination of metal with cardboard or with paper.

10. The drop-in probe according to claim 9, **characterised in that** the protective cap is formed from steel, preferably comprising a thickness of maximally 0.5 mm, or from a combination of zinc with cardboard or with paper.

11. The drop-in probe according to at least any one of claims 1 to 10, **characterised in that** the inlet opening is formed from quartz glass, wherein a deoxidizing means can be arranged therein.

12. The drop-in probe according to at least any one of claims 1 to 11, **characterised in that** it has a further temperature sensor.

13. The drop-in probe according to claim 12, **characterised in that** the further temperature sensor is arranged at the end of the measurement head located opposite the immersion end.

14. A method for determining phase transitions of a sample taken from a steel melt by thermal analysis by means of the drop-in probe according to any one of the preceding claims, wherein the parts of the signal cable as well as the metal tube (9), which at least partially surrounds the signal cable (8) and which are included in the total density of the measurement head of less than 7 g/cm³, are immersed into the steel melt and into a slag layer located on the steel melt in response to the operation of the drop-in probe.

15. The method according to the preceding claim, **characterised in that** the part of the signal cable, which is included in the total density of the measurement head of less than 7 g/cm³, is 50 cm long with an average cable density of approx. 1.6 g/cm³.

## Revendications

1. Sonde d'introduction pour la détermination de points de transition de phase d'un échantillon prélevé d'une coulée d'acier par analyse thermique, avec une tête de mesure (10) présentant une extrémité d'immersion dans laquelle sont disposés une chambre d'échantillon (11) présentant une ouverture d'entrée (15, 20) et un thermocouple (12) dépassant avec son joint brasé chaud dans la chambre d'échantillon, et qui présente un passe-câble pour un câble de signalisation (8) du thermocouple, dans laquelle le passe-câble sort de la tête de mesure par une ouverture de sortie à une extrémité de la tête de mesure opposée à l'extrémité d'immersion, et dans laquelle une droite entre l'extrémité d'immersion et l'ouverture de sortie forme un axe longitudinal de la tête de mesure, dans laquelle la tête de mesure présente entre son extrémité d'immersion et un plan coupant la droite perpendiculairement entre l'extrémité d'immersion et l'ouverture de sortie une densité d'au moins 7 g/cm³, que l'ouverture d'entrée débouche entre ce plan et l'extrémité d'immersion ou au niveau de l'extrémité d'immersion et que la densité totale de la tête de mesure se monte à moins de 7 g/cm³, **caractérisée en ce que** la chambre d'échantillon présente une ouverture de sortie gazeuse pour garantir un afflux régulier de la coulée d'acier liquide dans la chambre d'échantillon, dans laquelle l'ouverture de sortie gazeuse est fournie de sorte que de l'air refoulé par la coulée d'acier peut quitter la chambre d'échantillon à travers chaque ouverture de sortie gazeuse et faire ainsi de la place pour la coulée d'acier qui pousse par derrière, dans laquelle l'ouverture de sortie gazeuse est formée par une fente qui est disposée entre le câble de signalisation (8) et un tube métallique (9) qui protège le câble de signalisation d'un endommagement prématuré par la scorie et/ou la coulée d'acier.

2. Sonde d'introduction selon la revendication 1, **caractérisée en ce que** la densité totale de la tête de mesure y compris du tube métallique entourant au moins partiellement le câble de signalisation et de parties du câble de signalisation se monte à moins de 7 g/cm³.

3. Sonde d'introduction selon la revendication 1 ou 2, **caractérisée en ce qu'**un plan virtuel est à chaque fois formé perpendiculairement à l'axe longitudinal par le joint brasé chaud et par une partie de l'ouverture d'entrée disposée le plus loin de l'extrémité d'immersion, et que la tête de mesure une densité d'au moins 7 g/cm³ entre l'extrémité d'immersion et le plan le plus éloigné de l'extrémité d'immersion.

4. Sonde d'introduction selon une des revendications 1 à 3, **caractérisée en ce que** l'ouverture d'entrée présente un diamètre qui se monte au moins à un tiers du diamètre de la chambre d'échantillon et est au plus aussi grand que le diamètre de la chambre d'échantillon.

5. Sonde d'introduction selon au moins une des revendications 1 à 4, **caractérisée en ce que** le thermocouple s'étend approximativement parallèlement à l'axe longitudinal avec son joint brasé chaud en direction de l'extrémité d'immersion.

6. Sonde d'introduction selon au moins une des revendications 1 à 5, **caractérisée en ce que** le thermocouple est maintenu dans la chambre d'échantillon avec un matériau perméable au gaz.

7. Sonde d'introduction selon la revendication 6, **caractérisée en ce que** le passe-câble et l'ouverture de sortie sont reliés à la chambre d'échantillon par le biais du matériau perméable au gaz de manière perméable au gaz.

8. Sonde d'introduction selon au moins une des revendications 1 à 7, **caractérisée en ce qu'**un tube s'étendant de préférence de manière coaxiale à la droite, de préférence un tube métallique, à travers lequel les câbles de signalisation sont guidés, est disposé au niveau de l'ouverture de sortie.

9. Sonde d'introduction selon au moins une des revendications 1 à 8, **caractérisée en ce que** l'ouverture d'entrée présente sur son côté externe un capuchon de protection qui est formé de métal ou d'une combinaison de métal avec du carton ou avec du papier.

10. Sonde d'introduction selon la revendication 9, **caractérisée en ce que** le capuchon de protection est formé d'acier, de préférence avec une épaisseur de 0,5 mm maximum, ou d'une combinaison de zinc avec du carton ou avec du papier.

11. Sonde d'introduction selon au moins une des revendications 1 à 10, **caractérisée en ce que** l'ouverture d'entrée est formée de verre de quartz, dans laquelle un agent désoxydant peut être disposé en elle.

12. Sonde d'introduction selon au moins une des revendications 1 à 11, **caractérisée en ce qu'**elle présente un autre capteur de température.

13. Sonde d'introduction selon la revendication 12, **caractérisée en ce que** l'autre capteur de température est disposé à l'extrémité de la tête de mesure opposée à l'extrémité d'immersion.

14. Procédé de détermination de points de transition de phase d'un échantillon prélevé d'une coulée d'acier par analyse thermique avec la sonde d'introduction selon une des revendications précédentes, dans lequel les parties du câble de signalisation ainsi que le tube métallique (9) entourant au moins partiellement le câble de signalisation (8), qui sont incorporés dans la densité totale de la tête de mesure de moins de 7 g/cm³, sont immergés lors du fonctionnement de la sonde d'introduction dans la coulée d'acier et dans une couche de scorie se trouvant sur la coulée d'acier.

15. Procédé selon la revendication précédente, **caractérisé en ce que** la partie du câble de signalisation incorporée dans la densité totale de la tête de mesure de moins de 7 g/cm³ est de 50 cm de long à une densité de câble moyenne d'environ 1,6 g/cm³.
